# EUROPÄISCHE PATENTANMELDUNG

(11) **EP 0 637 456 A1**
(43) Veröffentlichungstag der Anmeldung: **08.02.1995**
(21) Anmeldenummer: 94110455.6
(22) Anmeldetag: 05.07.1994
(51) Int. Cl.: A61M 39/00, F16K 7/06

(54) **Schlauchklemme**

(30) Priorität: 09.07.1993 DE 9310226 U
(71) Anmelder: SPANG & BRANDS GmbH, D-61381 Friedrichsdorf (DE)
(72) Erfinder: Burkard, Hans, D-61440 Oberursel (DE)
(74) Vertreter: Jochem, Bernd, Dipl.-Wirtsch.-Ing.

(57) **Zusammenfassung**

Die Schlauchklemme dient zum wahlweisen Absperren und Öffnen von Schläuchen, z. B. Infusionsschläuchen. Sie hat die Form eines im wesentlichen U-förmigen Bügels aus Kunststoff mit verhältnismäßig steifen Schenkeln (10, 12), auf deren Innenseiten beim Abquetschen des Schlauchs zusammenwirkende Vorsprünge (16, 17; 18, 19) ausgebildet sind, sowie einem biegeelastischen Steg (14). Die freien Enden der Schenkel (10, 12) sind in Absperrstellung verrastbar, und ein Ende (20) ist ebenso wie der Steg (14) mit einem Loch zum Hindurchführen des Schlauchs versehen. Um Fehlbedienung durch Verkanten zu verhindern, ist vorgesehen, daß an beiden Seitenkanten eines Schenkels (10) oder an entgegengesetzten Seitenkanten beider Schenkel (10, 12) Randflansche (24) angeformt sind, zwischen denen sich beim Zusammendrücken die Vorsprünge (16, 18) spielfrei oder mit geringem Spiel befinden.

## Beschreibung

Die Erfindung betrifft eine Schlauchklemme zum wahlweisen Absperren und Öffnen von Schläuchen, z. B. Infusionsschläuchen, in Form eines im wesentlichen U-förmigen Bügels aus Kunststoff mit verhältnismäßig steifen Schenkeln, auf deren Innenseiten als Quetschkanten zusammenwirkende Vorsprünge ausgebildet sind, einem biegeelastischen Steg, durch welchen die Schenkel in eine divergierende Stellung aufspreizbar sind, und einer mit dem freien Ende des einen Schenkels verbundenen, zum zweiten Schenkel hin gebogenen, biegeelastisch auslenkbaren Federzunge mit einer einwärts weisenden Rastnase, hinter welcher beim Zusammendrücken der Schenkel das freie Ende des zweiten Schenkels in der Absperrstellung einrastbar ist, wobei der Steg und die Federzunge mit Löchern zum Hindurchführen des Schlauchs versehen und Mittel zur seitlichen Führung der Schenkel beim Zusammendrücken vorhanden sind.

Derartige Schlauchklemmen, jedoch ohne Mittel zur seitlichen Führung der Schenkel beim Zusammendrücken, sind seit langem Standardartikel, die zur üblichen Ausrüstung von Infusionsschläuchen gehören und daher von vornherein maschinell auf den Schläuchen montiert werden, bevor z. B. an deren einem Ende ein Besteck mit Nadel und am anderen Ende ein Anschlußteil angebracht werden. Grundsätzlich können solche Schlauchklemmen aber auch bei Schläuchen für andere medizinische und nicht medizinische Zwecke, z. B. in Laboratorien, Verwendung finden.

Die in Rede stehenden Schlauchklemmen sind funktional Zwei-Stellungs-Absperrorgane. In der offenen Stellung sitzen sie lose auf dem Schlauch und behindern den Durchfluß nicht. Drückt man die Schenkel bis zum Einrasten der Federzunge zusammen, behält die Schlauchklemme eine Absperrstellung, in welcher der Schlauch durch die inneren Vorsprünge der Schenkel dicht zusammengequetscht wird. Die Absperrstellung läßt sich leicht mit einer Hand lösen, indem die Federzunge mit Rastnase nach außen zurückgebogen wird, so daß sie den von ihr übergriffenen Schenkel wieder freigibt.

Die einfachen, nur wahlweise zu öffnenden und zu schließenden Schlauchklemmen sind sogar in solchen Fällen nützlich, wo ein einstellbares Absperrorgan, z. B. eine Rollenklemme, ein Hahn oder ein steuerbares Ventil vorhanden sind. Sie bieten dann nämlich die Möglichkeit, eine manchmal sorgfältig justierte Stellung des einstellbaren Absperrorgans mit einer bestimmten Durchflußrate unverändert beizubehalten, während der Schlauch abgesperrt und wieder geöffnet wird, so daß man den gewünschten Durchfluß nicht jedesmal neu einstellen muß.

Insbesondere die sog. Mini-Schlauchklemmen sind jedoch bei seitlichen Kräften verhältnismäßig labil, was auf das die Steifigkeit schwächende Loch im Steg des Bügels zurückzuführen ist. Es kann daher unter dem Zeitdruck des Krankenhausbetriebs vorkommen, daß sich die Schenkel beim Zusammendrücken verkanten, in dieser relativen Schrägstellung die Rastnase nicht richtig greift und dann später die Schlauchklemme, eventuell ausgelöst durch eine Bewegung des Patienten, von allein aufspringt. Diese Fehlfunktion kann bei bestimmten Infusionslösungen, die genau dosiert werden müssen, schwerwiegende Folgen haben. Um sie zu vermeiden, ist es bekannt, ein zusätzliches Teil in die Schlauchklemme einzusetzen, und zwar in den ungefähr zylindrischen Raum, der vom bogenförmigen Steg und den Vorsprüngen an den Innenseiten der Schenkel begrenzt wird. Das zusätzliche Teil hat im wesentlichen die Form eines längsgeteilten Zylinders mit einer mittleren radialen Durchgangsbohrung, durch welche der Schlauch hindurchgeführt werden muß, und mit im wesentlichen rechteckigen Endflanschen, die über die flache Seite des Halbzylinders vorstehen. Dieser Einsatz ist etwas länger als die Breite einer Schlauchklemme, so daß sich im montierten Zustand die Endflansche des Einsatzes außerhalb der Schlauchklemme befinden und eine seitliche Führung für deren Schenkel beim Zusammendrücken bilden. Im übrigen hat diese Konstruktion entscheidende Nachteile. Am stärksten fällt ins Gewicht, daß mit dem Einsatz ein zusätzliches, bewegliches Teil produziert und montiert werden muß. Dabei ist die Montage in der Schlauchklemme und auf dem Schlauch zu berücksichtigen. Bisher gibt es keine automatische Montage von Schlauchklemmen mit solchen Einsätzen, sie wäre auch sehr kompliziert und teuer. Darüber hinaus führen die über die Schlauchklemmen vorstehenden, einzeln beweglichen Einsätze zur Unsicherheit bei der Handhabung. Schließlich erschweren und begrenzen sie das Zusammendrücken der Schenkel, weil sie den vom Steg bogenförmig umgrenzten Raum weitgehend ausfüllen.

Der Erfindung liegt deshalb die Aufgabe zugrunde, eine Schlauchklemme der eingangs genannten Art zu schaffen, welche Fehlbedienungen und daraus folgende Fehlfunktionen mit sehr einfachen Mitteln wirksam verhindert, und diese Aufgabe wird erfindungsgemäß dadurch gelöst, daß an beiden Seitenkanten eines Schenkels oder an entgegengesetzten Seitenkanten beider Schenkel Randflansche mit solcher Höhe angeformt sind, daß sich beim Zusammendrücken, vor Erreichen der Raststellung die Vorsprünge spielfrei oder mit geringem seitlichen Spiel zwischen ihnen befinden.

Die vorgeschlagene Ausbildung der Schlauchklemmen mit seitlichen Randflanschen verteuert die Herstellung und Montage praktisch nicht, da der minimal größere Materialeinsatz nicht ins Gewicht fällt. Wichtig ist, daß die Montage der Schlauchklemmen auf dem Schlauch wie bisher automatisch erfolgen kann, wobei sich überraschend gezeigt hat, daß die Montage durch die Randflanschen sogar erleichtert wird, weil sich die Schlauchklemmen, die sich zunächst ungeordnet in einem Vorratsbehälter befinden, nicht mehr so leicht gegenseitig verhaken und daher ohne Schwierigkeiten maschinell vereinzeln lassen. Ein weiterer Vorteil besteht darin, daß die vorgeschlagenen Randflansche zur Steifigkeit wenigstens eines Schenkels der Schlauchklemme beitragen, während die elastischen Biegeeigenschaften des Stegs und der Federzunge unbeeinflußt bleiben.

Wegen der nunmehr verhältnismäßig großen seitlichen Flächen wird auch die Griffigkeit und Handhabung der Schlauchklemmen gefördert. Dabei macht es sich angenehm bemerkbar, wenn in bevorzugter Ausgestaltung der Erfindung die Breite zwischen den Außenflächen der Randflansche gleich der Breite der Aussenflächen der Schenkel, des Stegs und der Federzunge ist. Dann hat die Schlauchklemme den Charakter eines äußerlich glattflächigen, rundum weitgehend geschlossenen Kästchens, das sich sicher ergreifen, zwischen den Fingerspitzen halten sowie schließen und öffnen läßt.

Damit nicht ein Verkanten der Schenkel schon am Anfang des Zusammendrückens dazu führt, daß ein Vorsprung gegen einen Randflansch stößt und die weitere Schließbewegung der Schlauchklemme blockiert, ist in weiterer erfindungsgemäßer Ausgestaltung der Erfindung vorgesehen, daß wenigstens jeweils eine der beim Zusammendrücken der Schenkel aneinander vorbeibewegten Randkanten des Vorsprungs bzw. der Vorsprünge und der Randflansche angeschrägt ist. Die Schrägflächen leiten den Vorsprung bzw. die Vorsprünge sicher zwischen die Randflanschen.

Ein Ausführungsbeispiel der Erfindung wird nachstehend anhand der Zeichnung näher erläutert. Es zeigen:
- Fig. 1: einen Längsschnitt durch eine Schlauchklemme mit seitlichen Randflanschen;
- Fig. 2: eine Draufsicht auf die Schlauchklemme nach Fig. 1 von unten;
- Fig. 3: eine Endansicht der Schlauchklemme nach Fig. 1 mit Blickrichtung von rechts;
- Fig. 4: eine Endansicht der Schlauchklemme nach Fig. 1 mit Blickrichtung von links und
- Fig. 5: einen Querschnitt nach Schnittlinie V-V in Fig. 1.

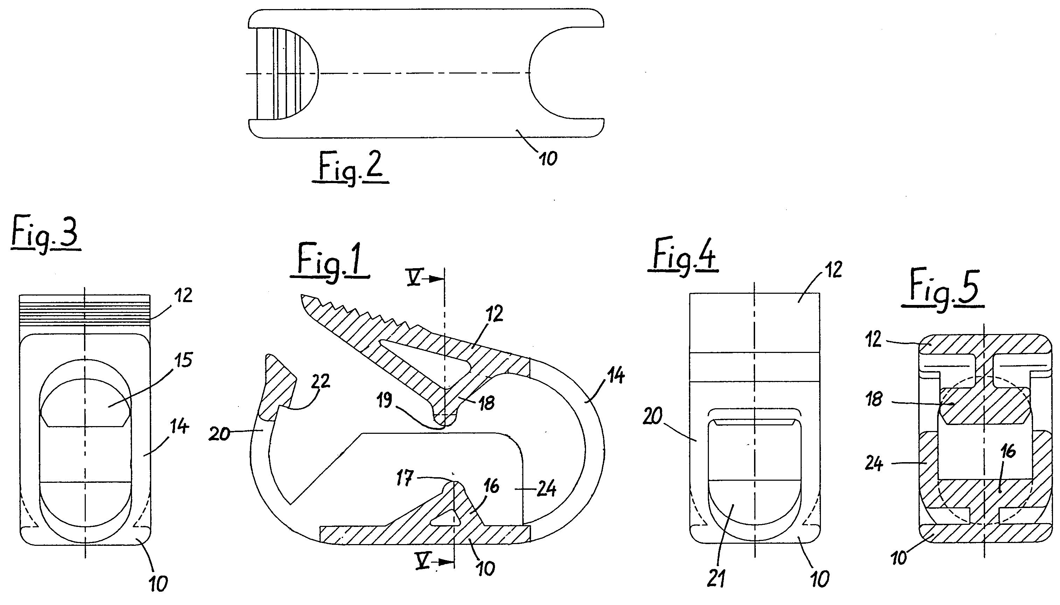

Die dargestellte Schlauchklemme hat im Querschnitt die aus Fig. 1 ersichtliche Form eines im wesentlichen U-förmigen Bügels mit einem ersten Schenkel 10, einem zweiten Schenkel 12 und einem die beiden Schenkel 10, 12 miteinander verbindenden, etwa halbkreisförmigen Steg 14. An den Innenseiten der Stege 10, 12 sind, ungefähr gegenüberliegend, Vorsprünge 16, 18 angeformt, die zusammenwirkende Quetschkanten 17 bzw. 19 bilden. Außerdem ist am freien Ende des ersten Schenkels 10 eine Federzunge 20 angeformt, die zum freien Ende des zweiten Schenkels 12 hin umgebogen ist und im geöffneten Zustand der Schlauchklemme eine solche Stellung einnimmt, daß der zweite Schenkel 12 bei Bewegung zum ersten Schenkel 10 hin gegen die schräge Endfläche der Federzunge 20 stößt und diese nach aussen zurückdrängt, bis er mit seinem freien Ende hinter einer am Ende der Federzunge 20 angeordneten, nach innen weisenden Rastnase 22 einrastet. Der Steg 14 und die ebenfalls bogenförmig gekrümmte Federzunge 20 sind mit Löchern 15 bzw. 21 versehen, um einen Schlauch in Längsrichtung der Schenkel 10, 12 durch die Schlauchklemme hindurchführen zu können. Durch Zusammendrücken der Schenkel 10, 12 wird der Schlauch zwischen den Quetschkanten 17 und 19 dicht zusammengequetscht, und die Schlauchklemme wird in dieser Absperrstellung verriegelt gehalten, wenn das freie Ende des zweiten Schenkels 12 hinter der Rastnase 22 der Federzunge 20 eingerastet ist. Zum Lösen der Verriegelung braucht nur das freie Ende der Federzunge 20 nach außen, d. h. mit Bezug auf Fig. 1 nach links, vom freien Ende des zweiten Schenkels 12 weg gedrückt zu werden.

Soweit bisher beschrieben, ist die gezeigte Schlauchklemme bekannt, so daß auf weitere Einzelheiten nicht näher eingegangen zu werden braucht.

Die Besonderheit der Schlauchklemme besteht darin, daß an dem mit der Federzunge 20 verbundenen ersten Schenkel 10 auf beiden Seiten Randflansche 24 angeformt sind. Im Beispielsfall erstrecken sie sich über die gesamte Länge des Schenkels 10. Auf die Länge der Randflansche 24 und die Form ihrer vorderen und hinteren Endkanten kommt es dabei jedoch nicht entscheidend an. Anders als in Fig. 1 gezeigt, könnte sich z. B. die stegseitige Endkante nicht quer, sondern schräg zur Längsrichtung des Schenkels 10 erstrecken, und die auf Seiten der Federzunge 20 gelegene Endkante könnte statt der gezeigten schrägen Lage quer zur Längsrichtung liegen, gerundet sein oder eine andere Form haben.

Wichtig ist hingegen, daß sich die Randflansche 24 im Bereich der Vorsprünge 16, 18, und zwar speziell der nach innen, im wesentlichen gegeneinander weisenden Quetschkanten 17, 19 angeordnet sind und wenigstens eine solche Höhe haben, daß sich der relativ zu den Randflanschen 24 bewegliche Vorsprung 18 mit seiner Quetschkante 19 spielfrei oder mit nur geringem seitlichen Spiel zwischen den beiden Randflanschen 24 befindet, wenn das freie Ende des Schenkels 12 hinter der Rastnase 22 einrastet. Man kann auch die Randflanschen 24 so hoch ausbilden, daß sich die relativ zu ihnen bewegliche Quetschkante 19 selbst im geöffneten Zustand der Schlauchklemme zwischen ihnen befindet. Für den praktischen Gebrauch wird man eine mittlere Höhe der Randflanschen 24 vorziehen, welche nicht ganz bis an die Lage der Quetschkante 19 im geöffneten Zustand der Schlauchklemme heranreicht, so daß in der Öffnungsstellung der Schlauch im Bereich zwischen den Quetschkanten 17, 19 teilweise sichtbar bleibt, aber beim Zusammendrücken der Schenkel 10, 12 alsbald die seitliche Führung des Vorsprungs 18 zwischen den Randflanschen 24 wirksam wird.

Die Randflansche 24 hätten einfach an die Seitenkanten einer bestehenden Schlauchklemme angeformt werden können. Sie würden dann gegenüber dem zweiten Schenkel 12 seitlich vorstehen. Um eine gleichmäßige äußere Form zu erhalten, wird es vorgezogen, die Breite der Vorsprünge 16 und 18 um soviel schmaler zu machen als die äußere Breite der Schenkel 10 und 12, daß die Außenflächen der Randflansche 24 jeweils bündig, d. h. in einer gemeinsamen Ebene mit den Seitenkanten beider Schenkel 10, 12 liegen, wie am besten aus Fig. 5 hervorgeht. Die gleichmäßige äußere Form gibt Sicherheit beim Hantieren mit den Schlauchklemmen, wobei man im Falle von Mini-Schlauchklemmen weitgehend auf das Fingerspitzengefühl angewiesen ist.

Als weitere Besonderheit zeigt Fig. 5, daß der Vorsprung 18 auf beiden Seiten zur Quetschkante 19 hin abgeschrägt ist. Dadurch zentriert sich der zweite Schenkel 12 beim Zusammendrücken der Schlauchklemme selbstätig zwischen den Randflanschen 24, auch wenn zu Beginn des Zusammendrückens die beiden Schenkel seitlich ein wenig verschoben bzw. verkantet sind. Alternativ oder zusätzlich könnten auch Schrägflächen an den inneren oberen Kanten der Randflansche 24 vorhanden sein.

## Patentansprüche

1. Schlauchklemme zum wahlweisen Absperren und Öffnen von Schläuchen, z. B. Infusionsschläuchen, in Form eines im wesentlichen U-förmigen Bügels aus Kunststoff mit verhältnismäßig steifen Schenkeln (10, 12), auf deren Innenseiten als Quetschkanten (17, 19) zusammenwirkende Vorsprünge (16, 18) ausgebildet sind, einem biegeelastischen Steg (14), durch welchen die Schenkeln (10, 12) in eine divergierende Stellung aufspreizbar sind, und einer mit dem freien Ende des einen Schenkels (10) verbundenen, zum zweiten Schenkel (12) hin gebogenen, biegeelastisch auslenkbaren Federzunge (20) mit einer einwärts weisenden Rastnase (22), hinter welcher beim Zusammendrücken der Schenkel (10, 12) das freie Ende des zweiten Schenkels (12) in der Absperrstellung einrastbar ist, wobei der Steg (14) und die Federzunge (20) mit Löchern (15, 21) zum Hindurchführen des Schlauchs versehen und Mittel zur seitlichen Führung der Schenkel (10, 12) beim Zusammendrücken vorhanden sind, **dadurch gekennzeichnet**, daß an beiden Seitenkanten eines Schenkels (10) oder an entgegengesetzten Seitenkanten beider Schenkel (10, 12) Randflansche (24) mit solcher Höhe angeformt sind, daß sich beim Zusammendrücken, vor Erreichen der Raststellung die Vorsprünge (16, 18) spielfrei oder mit geringem seitlichen Spiel zwischen ihnen befinden.

2. Schlauchklemme nach Anspruch 1, **dadurch gekennzeichnet**, daß die Randflansche (24) an dem ersten Schenkel (10) angeformt sind.

3. Schlauchklemme nach Anspruch 1 oder 2, **dadurch** **gekennzeichnet**, daß die Breite zwischen den Außenflächen der Randflansche (24) gleich der Breite der Außenflächen der Schenkel (10, 12), des Stegs (14) und der Federzunge (20) ist.

4. Schlauchklemme nach einem der Ansprüche 1 bis 3, **dadurch** **gekennzeichnet**, daß wenigstens jeweils eine der beim Zusammendrücken der Schenkel (10, 12) aneinander vorbeibewegten Randkanten des Vorsprungs (18) bzw. der Vorsprünge (16, 18) und der Randflansche (24) abgeschrägt ist.
